# EUROPEAN PATENT APPLICATION

(11) **EP 4 604 135 A1**
(43) Date of publication of application: **20.08.2025**
(21) Application number: 23876966.5
(22) Date of filing: 14.07.2023
(51) Int. Cl.: G16H 50/20

(54) **DIAGNOSIS SUPPORT METHOD, DIAGNOSIS SUPPORT PROGRAM, AND DIAGNOSIS SUPPORT SYSTEM**

(30) Priority: 14.10.2022 JP 2022165486
(71) Applicant: SHIMADZU CORPORATION, Kyoto-shi, Kyoto 604-8511 (JP)
(72) Inventor: HIROSE, Ryuta, Kyoto-shi, Kyoto 604-8511 (JP); HASEGAWA, Yukinori, Kyoto-shi, Kyoto 604-8511 (JP); NAKAYA, Tomohiro, Kyoto-shi, Kyoto 604-8511 (JP); UNO, Haruo, Kyoto-shi, Kyoto 604-8511 (JP); OYAMA, Shintaro, Nagoya-shi, Aichi 464-8601 (JP); NAKAGAWA, Yasunobu, Nagoya-shi, Aichi 464-8601 (JP); YAGI, Tetsuya, Nagoya-shi, Aichi 464-8601 (JP); IGUCHI, Mitsutaka, Nagoya-shi, Aichi 464-8601 (JP)
(74) Representative: Kilian Kilian & Partner mbB
(86) International application number: PCT/JP2023/026093
(87) International publication number: WO 2024/079957

(57) **Abstract**

A medical service support system obtains at least one item of patient information relating to a patient (steps S30 and S40) and inputs the at least one item to an estimation model and obtains candidates for a disease which may be affecting the patient from among a plurality of diseases (step S50). The medical service support system then obtains additional request information to additionally be obtained from the patient, based on the candidates for the disease (step S70). The medical service support system then presents the additional request information (step S90).

## Description

### TECHNICAL FIELD

The present invention relates to support of medical service.

### BACKGROUND ART

In particular in medium- to small-scale hospitals and clinics, an infection non-specialist may have to deal with an infection. It is difficult for the infection non-specialist to be conversant with all infections, and needs for medical service support by an infection specialist are great. With increase in drug-resistant pathogens, importance of proper use of antimicrobial drugs has increasingly been recognized. Expectation for the medical service support to the infection non-specialist is great also in this regard.

Infection specialists conversant in infection medical care are definitely lacking as compared with the number of medical institutions, and demands for infection medical service support tools are great. A system using a database in which information on infections is integrated has conventionally been proposed as an exemplary infection medical service support tool (see NPL 1). In this system, when patient information such as coughing or fever is inputted, a list of infections which may be affecting a patient is shown and information on the infections is also provided together.

### CITATION LIST

### NON PATENT LITERATURE

NPL 1: "Advancing the global effort against Infectious Diseases," [online], [Searched on May 10, 2022], the Internet <URL: https://www.gideononline.com/>

### SUMMARY OF INVENTION

### TECHNICAL PROBLEM

According to the system above, even a non-specialist of a given disease such as an infection can know candidates for the disease affecting the patient, and the system facilitates medical service.

Information on the disease outputted in the system, however, is identified based on limited patient information, and there is a need for a system capable of outputting more reliable information.

The present invention was made in view of such circumstances, and an object thereof is to provide a technique to support medical service of a disease to a non-specialist of the disease.

### SOLUTION TO PROBLEM

A medical service support method according to one aspect of the present disclosure includes obtaining at least one item of patient information relating to a patient, inputting the at least one item to an estimation model and obtaining candidates for a disease which may be affecting the patient from among a plurality of diseases, obtaining additional request information to additionally be obtained from the patient, based on the candidates for the disease, and presenting the additional request information.

A medical service support program according to one aspect of the present disclosure, by being executed by at least one processor of a computer, causes the computer to perform the medical service support method described above.

A medical service support system according to one aspect of the present disclosure includes a processor, a communication interface, and a storage where an estimation model is stored, the estimation model being configured to output candidates for a disease which may be affecting a person associated with patient information among a plurality of diseases, in response to input of at least one item of the patient information, the processor obtains input of a value of the at least one item of the patient information relating to a patient, inputs the value of the at least one item to the estimation model to obtain candidates for the disease which may be affecting the patient, and obtains additional request information to additionally be obtained from the patient, based on the candidates for the disease, and the communication interface presents the additional request information.

### ADVANTAGEOUS EFFECTS OF INVENTION

According to one aspect of the present disclosure, a non-specialist of a disease is provided with suggestion about a value of which item he/she should further consider, as additional request information. Support for medical service of a disease is thus provided to a non-specialist of the disease.

### BRIEF DESCRIPTION OF DRAWINGS

Fig. 1 is a diagram for illustrating provision of information by a medical service support system 100.
Fig. 2 is a diagram showing exemplary generated structured data.
Fig. 3 is a diagram showing a hardware configuration of medical service support system 100.
Fig. 4 is a diagram showing a mathematical expression showing an exemplary estimation model.
Fig. 5 is a flowchart of a process performed for presenting to a primary doctor, candidates for an infection which may be affecting a patient.
Fig. 6 is a diagram showing an exemplary initial screen.
Fig. 7 is a diagram showing an exemplary screen that shows electronic medical chart data inputted by the primary doctor.
Fig. 8 is a diagram showing an exemplary screen updated as instructed in step S90.
Fig. 9 is a diagram showing an exemplary screen to which new physical finding has been inputted.
Fig. 10 is a diagram showing an exemplary new display screen.

### DESCRIPTION OF EMBODIMENTS

An embodiment of the present disclosure will be described in detail below with reference to the drawings. The same or corresponding elements in the drawings have the same reference characters allotted and description thereof will not be repeated.

### [Overall Configuration]

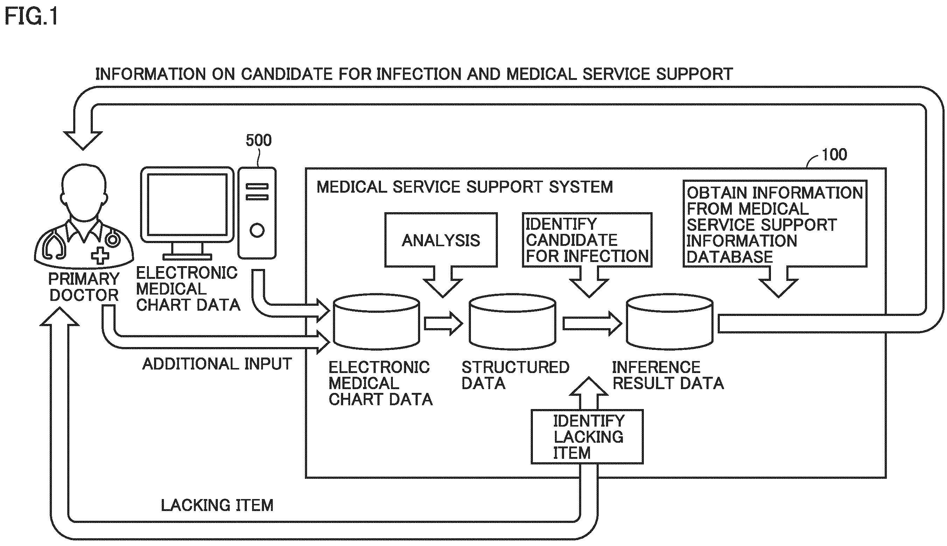

Fig. 1 is a diagram for illustrating provision of information by a medical service support system 100. In one implementation, the medical service support system according to the present disclosure provides information on medical service and/or therapy of a disease. An "infection" will be described below by way of example of a disease. The disease of interest in the present disclosure is not limited to the infection but may be a non-infection.

In a situation as shown in Fig. 1, a primary doctor is an infection non-specialist who sees a patient unclear as to whether or not he/she is affected by an infection, and the primary doctor operates an information terminal 500. Information terminal 500 can communicate with medical service support system 100.

When medical service support system 100 receives input of electronic medical chart data from information terminal 500, it generates structured data by analyzing the electronic medical chart data. The structured data includes at least one item relating to a patient and a value of the at least one item.

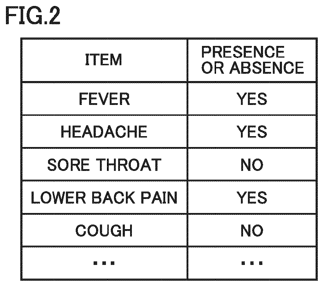

Fig. 2 is a diagram showing exemplary generated structured data. In the example shown in Fig. 2, the structured data includes at least five items. The structured data includes values of five respective items shown in Fig. 2.

An exemplary item is "fever" and an exemplary value is "YES" for the item "fever". In one implementation, the value of the item "fever" in the structured data for one patient is "YES" when the patient's body temperature is equal to or higher than 37.0°C, and the value is "NO" when the body temperature is lower than 37.0°C.

Referring back to Fig. 1, medical service support system 100 uses at least one value in the structured data to identify candidates for an infection which may be affecting a patient. Medical service support system 100 obtains medical service reference information about each of the identified candidates for the infection from a medical service support information database. Medical service support system 100 then transmits the candidates for the infection and the medical service reference information for each candidate to information terminal 500.

Medical service support system 100 identifies as a "lacking item," an item a value of which is required for improvement in accuracy of the identified candidate. Medical service support system 100 identifies the lacking item based on the structured data and then transmits the identified lacking item to information terminal 500.

Information terminal 500 shows the lacking item. In response, the primary doctor inputs the value of the lacking item to information terminal 500. The value of the lacking item is thus added to the electronic medical chart data.

Information terminal 500 transmits the electronic medical chart data to which the value of the lacking item has been added to medical service support system 100. Medical service support system 100 uses the electronic medical chart data to which the value of the lacking item has been added to generate the structured data, and uses the generated structured data to identify again candidates for an infection which may be affecting the patient.

### [Hardware Configuration]

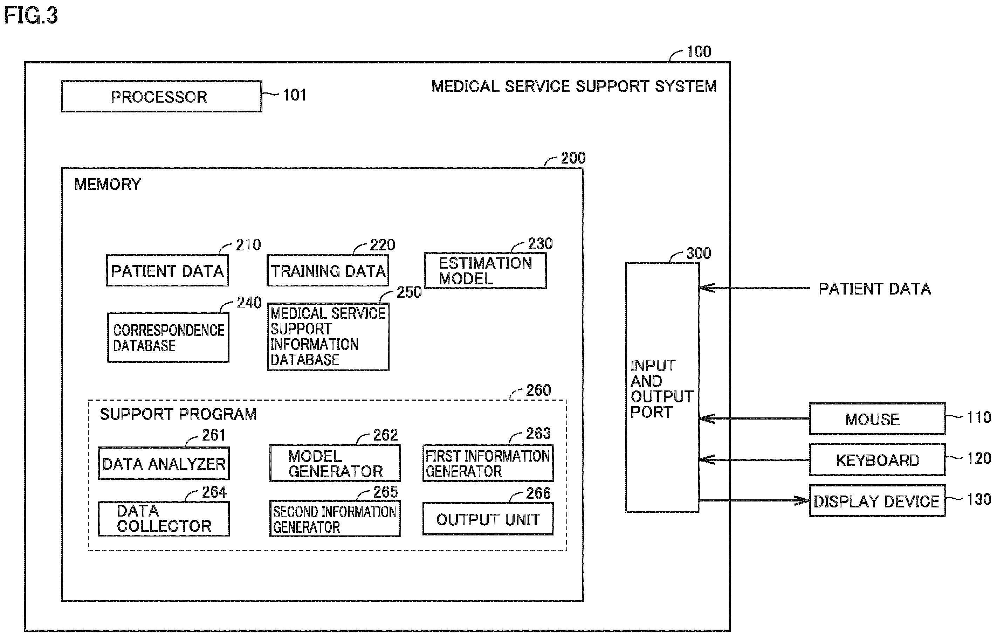

Fig. 3 is a diagram showing a hardware configuration of medical service support system 100.

Medical service support system 100 is configured, for example, based on a personal computer. Medical service support system 100 may be configured with a server accessible from at least one information terminal over a network such as the Internet.

Medical service support system 100 includes a processor 101, a memory 200, and an input and output port 300. A mouse 110, a keyboard 120, and a display device 130 are connected to input and output port 300.

Input and output port 300 may be a communication interface for data communication over the network. Patient data is inputted to input and output port 300. The patient data is, for example, electronic medical chart data. In one implementation, the structured data is generated from the electronic medical chart data. The structured data may be inputted to input and output port 300. Specifically, the primary doctor may extract a value of the at least one item included in the structured data from the electronic medical chart data and input the value of the at least one item to input and output port 300 through information terminal 500.

Patient data 210, training data 220, an estimation model 230, a correspondence database 240, a medical service support information database 250, and a support program 260 are stored in memory 200.

Training data 220 is data to be used for machine learning by estimation model 230, training data 220 relating to a plurality of persons. **In** training data 220, the structured data as described with reference to Fig. 2 for each of the plurality of persons is tagged with infection affection information of each person. The affection information includes presence or absence of affection by an infection and a type of the affecting infection.

Estimation model 230 is a program for performing calculation in accordance with a model. Though an exemplary algorithm of estimation model 230 is logistic regression, the algorithm applied in estimation model 230 is not limited thereto. In one implementation, estimation model 230 is prepared for each of at least two types of infections, and the estimation model for each infection derives whether or not a patient is affected by the infection. The estimation model for each infection may derive a probability (certainty).

Correspondence database 240 is exemplary correspondence information, and brings each of at least two types of infections in correspondence with items to be used for estimation by estimation model 230 (at least one item included in the structured data). For example, correspondence database 240 brings an infection "pyelonephritis" in correspondence with three types of items (fever, frequent urination, and flank pain).

Medical service support information database 250 brings each of the at least two types of infections in correspondence with medical service support information for each infection (information for support to doctors in medical service). The medical service support information is exemplary additional information of the infection.

Support program 260 includes, as its function, a data analyzer 261, a model generator 262, a first information generator 263, a data collector 264, a second information generator 265, and an output unit 266. **In** one implementation, each of functions of data analyzer 261, model generator 262, first information generator 263, data collector 264, second information generator 265, and output unit 266 is performed by execution of a given program by processor 101.

Data analyzer 261 creates the structured data as described with reference to Fig. 2 from patient data 210.

**In** one implementation, data analyzer 261 extracts a portion in a pre-registered pattern from text of electronic medical chart data stored as patient data 210 by applying analysis processing such as morphemic analysis to the text. The "pre-registered pattern" refers to a pattern corresponding to a value of the structured data. Data analyzer 261 then recognizes the meaning of the portion in the extracted pattern in the electronic medical chart data and identifies a value corresponding to the recognized meaning. Data analyzer 261 then generates the structured data by combining the at least one identified value.

An exemplary registered pattern is "the body temperature is xx °C" and a portion "xx" means a numerical value. When the electronic medical chart data includes text "the body temperature is 37.5°C," data analyzer 261 extracts the text "the body temperature is 37.5°C" as the portion in the pre-registered pattern. Data analyzer 261 then recognizes that the body temperature of the patient is 37.5°C as the meaning of the text. Data analyzer 261, on the other hand, is set such that the item and the value thereof in the structured data are identified based on the recognized meaning. For example, setting is made such that when the body temperature is equal to or higher than 37.0°C, the item "fever" and the value thereof "YES" are identified, and when the body temperature is lower than 37.0°C, the item "fever" and the value thereof "NO" are identified. Thus, when the electronic medical chart data includes the text "the body temperature is 37.5°C," data analyzer 261 generates the structured data including the value "YES" of the item "fever".

The item of the structured data includes at least one of patient interview information, physical finding, and an examination result. The patient interview information, the physical finding, and the examination result may be included in the electronic medical chart data.

The patient interview information includes information obtained from a patient in an interview with the patient by the primary doctor. The patient interview information may include information obtained from the patient in an interview with the patient by another doctor in the past. Exemplary items included in the patient interview information include a main complaint of the patient.

The physical finding includes information collected from the patient by the primary doctor in medical examination (visual examination, auscultation, palpation, percussion, tendon reflex, light reflex, or the like).

The examination result includes a result of an examination conducted on the patient (a blood test, computed tomography (CT) scan, body temperature measurement, weight measurement, blood pressure measurement, heart rate measurement, or the like).

The item of the structured data may further include basic information of the patient (age, sex, a degree of necessity of care, a history of smoking, or the like), allergy information, and/or an item relating to anamnesis.

Model generator 262 uses training data 220 to perform machine learning processing on estimation model 230. Estimation model 230 subjected to machine learning processing is herein also referred to as a "trained model."

First information generator 263 derives a result of determination as to an infection that may be affecting the patient by applying the structured data generated from the patient data to the trained model. In one implementation, first information generator 263 uses the trained model for each type of the infection to derive the probability (certainty) that the patient is affected by each infection, and identifies as a candidate for the infection, the infection the probability of which is equal to or larger than a given value.

Data collector 264 collects medical service support information for each of the candidates identified by first information generator 263, from medical service support information database 250.

Second information generator 265 identifies as the lacking item, an item a value of which is lacking in the structured data generated from patient data 210, for each of the candidates identified by first information generator 263, by referring to correspondence database 240.

Output unit 266 generates image information for showing information identified in support program 260 and instructs information terminal 500 to show the image information.

### [Identification of Candidate for Infection]

One implementation of identification of a candidate for an infection by first information generator 263 will be described. As described above with reference to Fig. 3, in one implementation, estimation model 230 is prepared for each type of the infection and first information generator 263 derives the probability that the patient is affected by each infection by applying the structured data to each estimation model 230.

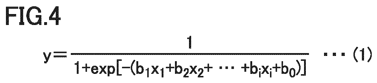

Fig. 4 is a diagram showing a mathematical expression showing an exemplary estimation model. An expression (1) shown in Fig. 4 complies with logistic regression analysis. In the expression (1), a subscript represents the type of the item included in the structured data. In the expression (1), i types of items from "1" to "i" are defined. A value of "i" may be different depending on the type of the infection. Specifically, the type and the number of items to be used for estimation of the probability of affection by each infection may be different for each infection. In correspondence database 240, each infection is associated with i types of items included in the expression (1) prepared for each infection.

In the expression (1), x represents an explanatory variable and corresponds to the value of each item in the structured data. For example, when the value is "YES" for the item "fever", x is 1, and when the value is "NO", x is 0.

In the expression (1), b represents a coefficient. A value of each of b₀ to bᵢ is set in machine learning for each infection.

In the expression (1), y represents an objective variable and represents a result of calculation for each infection. The value of y represents certainty of affection by each infection.

First information generator 263 derives certainty that the patient is affected by each infection by application of the value of each item included in the structured data to the estimation model for each infection.

First information generator 263 then identifies the infection the value of "certainty" of which is equal to or larger than a given threshold value, as a candidate for the infection that is affecting the patient.

A manner of identification of the candidate for the infection described with reference to Fig. 4 is merely by way of example. The candidate for the infection may be identified in any other type of manner such as multivariable analysis where at least one type included in the structured data is defined as the variable.

In application of the structured data to the estimation model, in connection with at least one infection, the value of at least one of items defined in the estimation model may not be included in the structured data. In other words, at least one of the items defined in the estimation model may be missing in the structured data.

An example in which the estimation model for an infection "pyelonephritis" defines three types of items (fever, frequent urination, and flank pain) is assumed. When the primary doctor incorporates information on fever and frequent urination but does not incorporate information on flank pain in electronic medical chart data, the structured data includes values of the item "fever" and the item "frequent urination" but does not include the value of the item "flank pain." In such a case, first information generator 263 performs missing value processing in derivation of certainty with the use of the estimation model. Exemplary missing value processing is random complement of the value of the missing item with a value of another item to be used in the expression of the same estimation model. Another example is complement of the value of the missing item with an average value of values of other items to be used in the expression of the same estimation model. For example, when the value of the item "flank pain" is missing, this value is complemented by the average value of the value of the item "fever" and the value of the item "frequent urination."

### [Identification of Lacking Item]

Second information generator 265 determines whether or not there is a missing item as above, for each of the infections identified as the candidates by first information generator 263. More specifically, when there is an item not included in the structured data among items associated with the infection in correspondence database 240 for each of the infections identified as the candidates by first information generator 263, second information generator 265 determines that there is a missing item. Second information generator 265 determines that there is no missing item when all of the items associated with the infection in correspondence database 240 are included in the structured data.

When there is a missing item, second information generator 265 then identifies that item as the lacking item.

### [Important Factor]

In memory 200, a score of importance of each item calculated at the time of generation of the estimation model may be stored for each infection. Second information generator 265 may identify an item having the score of the importance equal to or larger than a given value among items included in the structured data, as the important factor for each infection.

"Being equal to or larger than a given value" may mean that the score of the importance has a "positive" value equal to or larger than a certain value.

"Being equal to or larger than a given value" may mean that an absolute value of the score of the importance is equal to or larger than a given value. When the score of the importance of a certain item has a positive value, magnitude of the absolute value of the score represents likelihood of infection. When the score of the importance of a certain item has a negative value, magnitude of the absolute value of the score represents unlikelihood of infection. When "being equal to or larger than a given value" means that the absolute value of the score of the importance is equal to or larger than a given value, the item identified as the important factor characterizes likelihood of infection or unlikelihood of infection.

### [Flow of Process]

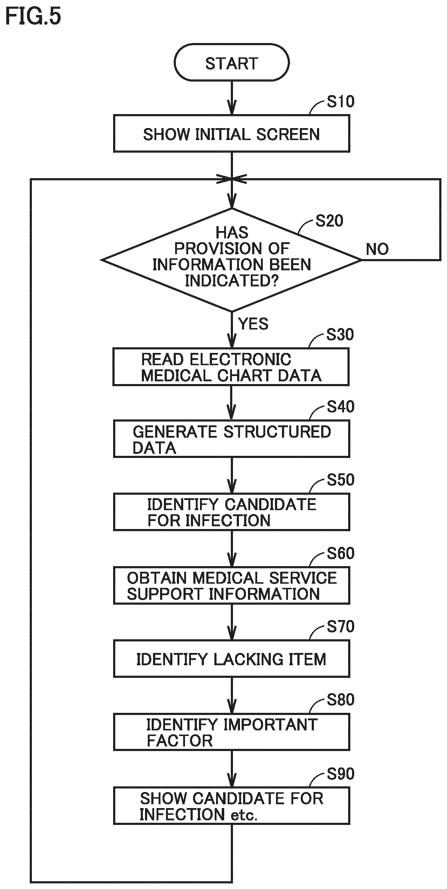

Fig. 5 is a flowchart of a process performed for presenting to the primary doctor, candidates for an infection that may be affecting a patient. In one implementation, the process in Fig. 5 is performed by execution of a given program (medical service support program) by processor 101 of medical service support system 100. The process in Fig. 5 will be described below. In one implementation, the process in Fig. 5 is started in response to a request from information terminal 500 to medical service support system 100 to start provision of information. In one implementation, information terminal 500 requests medical service support system 100 to start provision of information in response to start-up of an application program for medical service support and an operation to request start of provision of information in the application program.

In step S10, medical service support system 100 instructs information terminal 500 to show an initial screen. Information terminal 500 thus shows the initial screen on the display thereof.

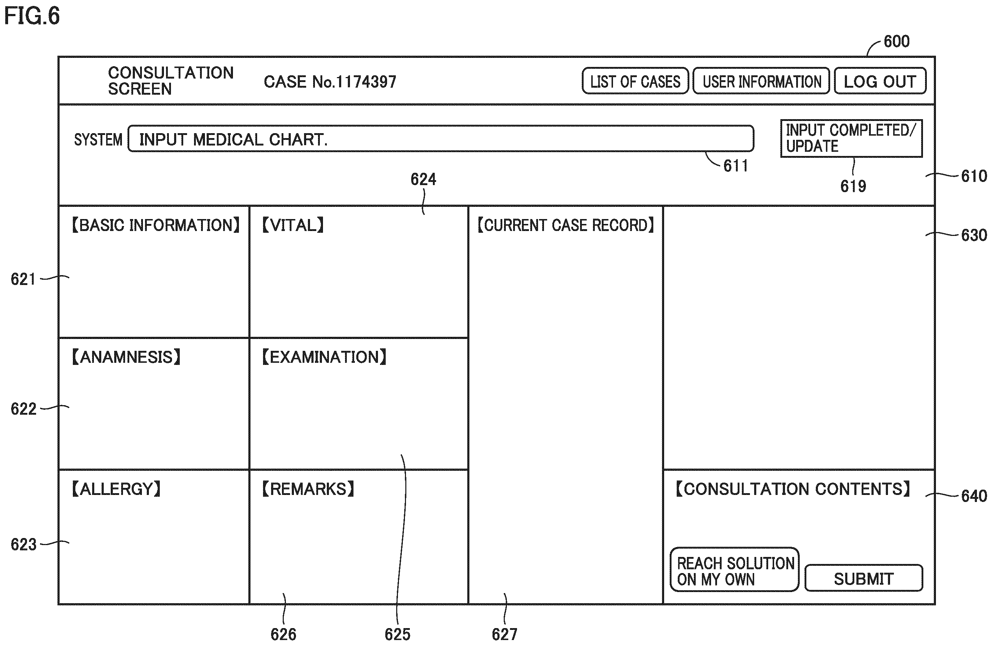

Fig. 6 is a diagram showing an exemplary initial screen. In Fig. 6, a screen 600 includes a first communication frame 610, fields 621 to 627 and 630, and a second communication frame 640.

First communication frame 610 shows information for communication by the primary doctor with medical service support system 100. First communication frame 610 includes a field 611 and a button 619. In the example in Fig. 6, a message (input medical chart) to the primary doctor is shown in field 611. Button 619 is operated to instruct medical service support system 100 to provide information for data inputted in the screen.

Electronic medical chart data of a patient is inputted in each of fields 621 to 627. More specifically, basic information (age, sex, a degree of necessity of care, a history of smoking, or the like) of the patient is inputted in field 621. Anamnesis of the patient is inputted in field 622. Allergy information of the patient is inputted in field 623. Vital information among results of examinations conducted on the patient is inputted in field 624. A result of an examination (a result of a blood test, a result of CT scan, or the like) other than the vital information among the results of examinations conducted on the patient is inputted in field 625. Physical finding is inputted in field 626. Patient interview information (a main complaint or the like) is inputted in field 627. A manner of input of the electronic medical chart data in the description above is merely by way of example. Medical service support system 100 may receive input of the electronic medical chart data described in a text format and may generate screen data as shown in Fig. 6 by extracting data to be shown in fields 621 to 627 from the electronic medical chart data.

In field 630, information on candidates for an infection that may be affecting the patient is shown, the information being provided from medical service support system 100 to the primary doctor.

Second communication frame 640 is used for the primary doctor to interact with an infection specialist.

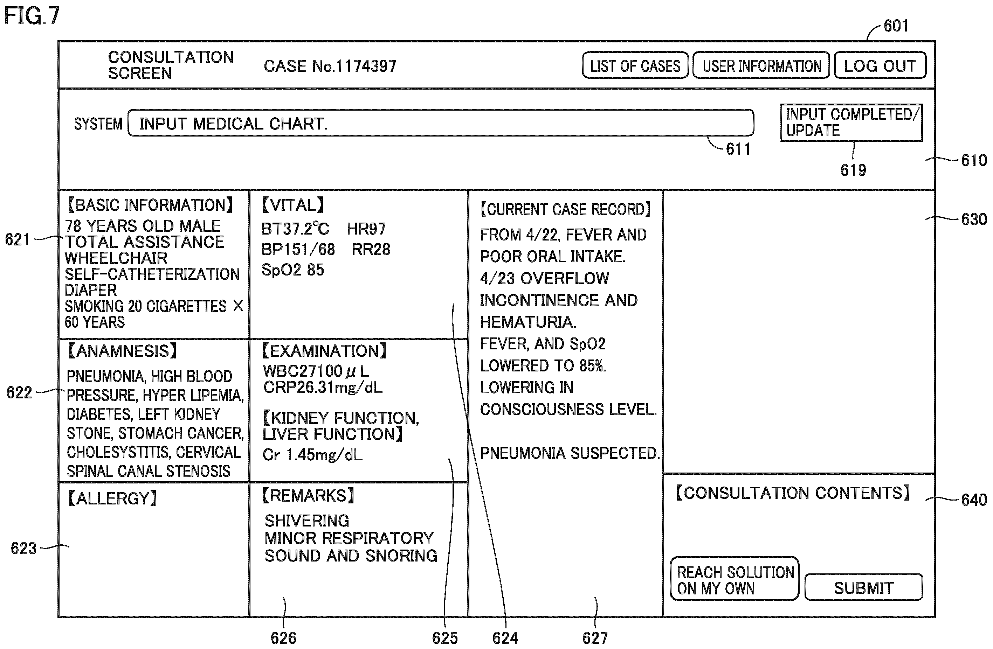

The primary doctor who visually recognizes screen 600 inputs the electronic medical chart data in fields 621 to 627. Fig. 7 is a diagram showing an exemplary screen that shows the electronic medical chart data inputted by the primary doctor. In Fig. 7, a screen 601 shows the electronic medical chart data inputted by the primary doctor in each of fields 621 to 627.

Thereafter, the primary doctor operates button 619. The inputted electronic medical chart data is thus transmitted from information terminal 500 to medical service support system 100.

Referring to back to Fig. 5, in step S20, medical service support system 100 determines whether or not it has been instructed to provide information by information terminal 500. Medical service support system 100 repeats control in step S20 until it determines that it has been instructed to provide information (NO in step S20). When medical service support system 100 determines that it has been instructed to provide information (YES in step S20), it allows control to proceed to step S30.

In step S30, medical service support system 100 reads the electronic medical chart data transmitted from information terminal 500.

In step S40, medical service support system 100 generates the structured data as described with reference to Fig. 2 by using the electronic medical chart data read in step S30.

In step S50, medical service support system 100 identifies at least one candidate for an infection that may be affecting the patient by using the structured data generated in step S40.

In step S60, medical service support system 100 obtains medical service support information for each of the at least one candidate for the infection identified in step S50, by referring to medical service support information database 250.

In step S70, medical service support system 100 identifies the lacking item for each of the at least one candidate for the infection identified in step S50.

In step S80, medical service support system 100 identifies the important factor for each of the at least one candidate for the infection identified in step S50.

In step S90, medical service support system 100 uses results in steps S50 to S80 to generate a display screen to show information on candidates for the infection or the like and instructs information terminal 500 to show the generated display screen. The display screen is thus updated in information terminal 500. Thereafter, medical service support system 100 has control return to step S20.

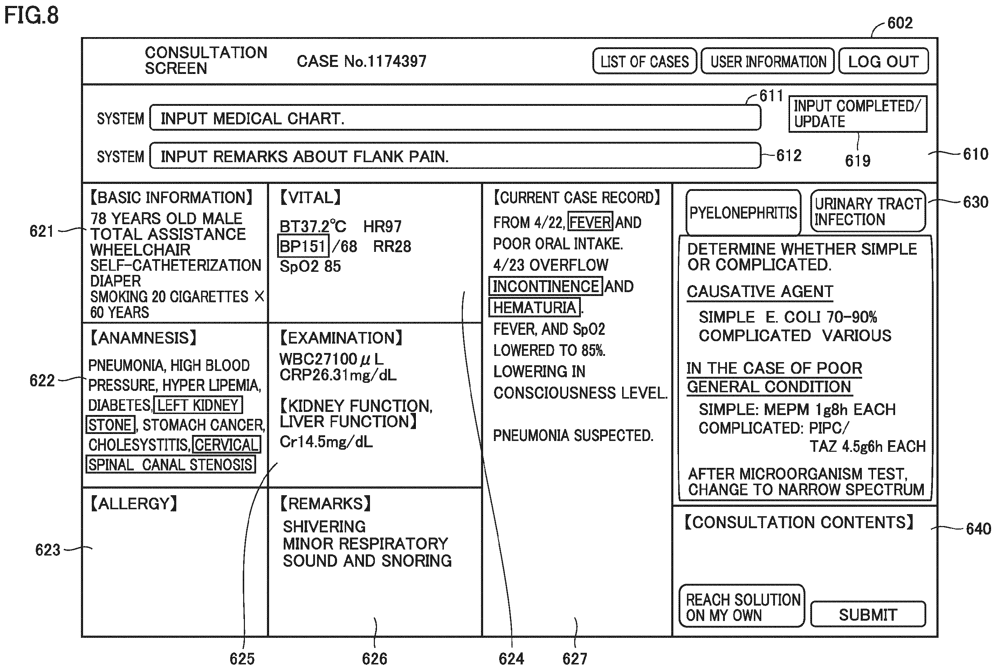

Fig. 8 is a diagram showing an exemplary screen updated as instructed in step S90. In Fig. 8, a screen 602 is an exemplary screen after update of screen 601 in Fig. 7.

As compared with screen 601 in Fig. 7, in screen 602 in Fig. 8, at least one candidate for the infection that may be affecting the patient is shown in field 630. At least one tab shown in field 630 corresponds to at least one respective candidate for the infection identified in step S50.

In field 630 in Fig. 8, two tabs showing two respective types of infections (pyelonephritis and urinary tract infection) are shown.

More specifically, in field 630, two tabs are shown to switch contents to be shown. Fig. 8 shows a state in which, of the tab of "pyelonephritis" and the tab of "urinary tract infection," contents in the tab "pyelonephritis" are shown. The primary doctor can have contents in the tab of "urinary tract infection" shown in field 630 by operating information terminal 500.

Contents in each tab in field 630 are medical service support information of a corresponding infection. Medical service support information of the candidate for each infection obtained in step S60 is shown in each tab in field 630.

As compared with Fig. 7, a field 612 is added to first communication frame 610 in Fig. 8. When the lacking item is identified in step S70, medical service support system 100 adds a field to first communication frame 610 and shows a message encouraging input of a value of the lacking item to the added field. The field may be added for each lacking item. For example, when two types of candidates are identified and one lacking item is identified for each of the two types of candidates, two fields may be added. A message encouraging input of a value of the lacking item identified for the first candidate is shown in one of the two added fields, and a message encouraging input of a value of the lacking item identified for the second candidate may be shown in remaining one of the two added fields.

In the example in Fig. 8, a message "input remarks about flank pain" is shown in field 612. This message corresponds to identification of "flank pain" as the lacking item. More specifically, medical service support system 100 has this message shown to encourage the primary doctor to further input the value of the identified lacking item (as physical finding).

In the example in Fig. 8, as compared with Fig. 7, a part of text shown in fields 621 to 627 is shown as being emphasized by being enclosed by a frame. The text shown as being emphasized corresponds to the important factor identified in step S80. In one implementation, the important factor is shown as being emphasized, as a basis of identification as the candidate in connection with the infection identified as the candidate.

More specifically, medical service support system 100 may cause a portion corresponding to the important factor identified in step S80 in the text (electronic medical chart data) shown in fields 621 to 627 to be shown as being emphasized. The portion corresponding to the important factor is a portion of the electronic medical chart data used for generation of the value of the item identified as the important factor in generation of the structured data.

An example in which the infection "pyelonephritis" is identified as the candidate, correspondence database 240 defines three types of items (fever, frequent urination, and flank pain) as the items to be used for estimation of the infection "pyelonephritis," and "fever" and "frequent urination" among them are identified as the important factors is assumed. In this case, the structured data further includes the value "YES" of the item "fever" generated from text "BT 37.2°C" in the electronic medical chart data and the value "YES" of the item "frequent urination" generated from text "increase in number of times of discharge of urine" in the electronic medical chart data.

In the above case, a portion corresponding to the important factor "fever" in the electronic medical chart data is "BT 37.2°C" and a portion corresponding to the important factor "frequent urination" is "increase in number of times of discharge of urine." Therefore, in the case above, in fields 621 to 627, "BT 37.2°C" and "increase in number of times of discharge of urine" in the electronic medical chart data are shown as being emphasized.

In the process in Fig. 5 described above, when the primary doctor inputs the electronic medical chart data of the patient, candidates for an infection which may be affecting the patient are presented to the primary doctor, and an item which may be lacking for identification of the candidates as the infection which is affecting the patient is presented as the message encouraging input of the value of the lacking item. Thus, suggestions as to the type of items required for using the presented candidates for final diagnosis are provided to the primary doctor as the lacking item.

The primary doctor may additionally input the value of the lacking item upon looking at the message. Medical service support system 100 may use the initially inputted value and the additionally inputted value of the lacking item to identify again candidates for the infection with which the patient may be infected.

More specifically, as medical service support system 100 indicates update of the screen in step S90, information terminal 500 shows the message encouraging input of the value of the lacking item as shown as field 612 in Fig. 8. The primary doctor inputs new physical finding to the screen in accordance with the message shown in field 612.

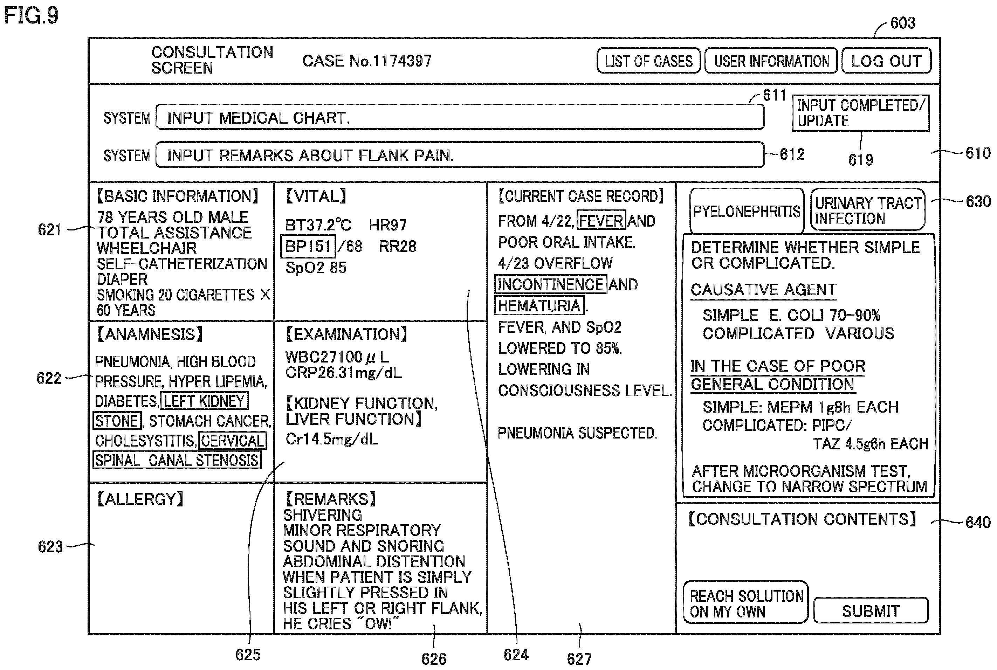

Fig. 9 is a diagram showing an exemplary screen to which new physical finding has been inputted. A screen 603 in Fig. 9 further includes in field 626, text "abdominal_distention_when patient is simply slightly pressed in his left or right flank, he cries 'ow!‴ corresponding to the new physical finding, as compared with screen 602 in Fig. 8. The primary doctor thereafter operates button 619.

Medical service support system 100 indicates update of the screen in step S90, and thereafter has control return to step S20. In response to operation on button 619 by the primary doctor, medical service support system 100 determines that it has been instructed to provide information (YES in step S20) and has control proceed to step S30.

In step S30, medical service support system 100 reads the electronic medical chart data including the new physical finding. Then in step S40, medical service support system 100 uses the electronic medical chart data read in step S30 to generate new structured data. Medical service support system 100 then uses in step S50, the new structured data to identify candidates for an infection, obtains in step S60, medical service support information of the candidate identified in step S50, identifies in step S70, the lacking item for the candidate identified in step S50, and identifies in step S80, the important factor of the candidate identified in step S50. Medical service support system 100 then uses in step S90, results in steps S50 to S80 performed after it reads the electronic medical chart data including the new physical finding, to generate a new display screen, and instructs information terminal 500 to show the new display screen. The display screen is thus updated in information terminal 500.

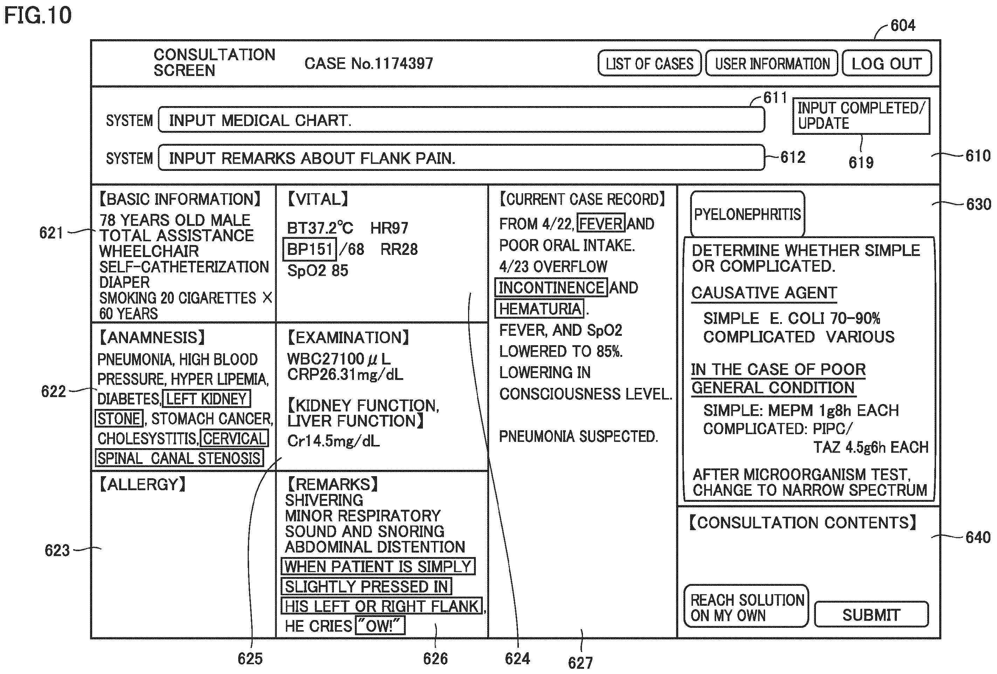

Fig. 10 is a diagram showing an exemplary new display screen. In a screen 604 in Fig. 10, a field 630 includes only "pyelonephritis" as the candidate for the infection as compared with screen 603 in Fig. 9. In screen 604 in Fig. 10, the text "when patient is simply slightly pressed in his left or right flank, he cries 'ow!‴ in field 626 is added as the emphasized text, as compared with screen 603 in Fig. 9.

In the example described with reference to Figs. 9 and 10, with addition of the text "when patient is simply slightly pressed in his left or right flank, he cries 'ow!'," the value "YES" of the item "flank pain" is added to the structured data, and lowering in certainty of the urinary tract infection of the two candidates (pyelonephritis and urinary tract infection) shown in Fig. 8 has occurred. In other words, in this example, as a result of the value of the lacking item, the number of candidates for the infection has decreased. The primary doctor can thus be provided with more accurate information as the information to be provided as the candidate for the infection with which the patient may be infected.

In the present embodiment, medical service support information for each candidate is further provided. The primary doctor can thus be provided with useful information in medical service for the patient even when the primary doctor is not the infection specialist.

In the present embodiment, for each candidate, the portion corresponding to the important factor in the electronic medical chart data is presented as emphasized representation. The primary doctor can thus be provided with useful information in making final diagnosis by using the presented candidate.

Though medical service support information database 250 is stored in memory 200 of medical service support system 100 in the present embodiment, it may be stored in a storage other than medical service support system 100. Medical service support system 100 may obtain medical service support information of each of at least one candidate for the infection by accessing the storage.

### (Modification)

In the present embodiment, the medical service support system that identifies the lacking item that allows improvement in accuracy of candidates for an infection when it is inputted to the estimation model and presents the lacking item to the primary doctor is described. In the present embodiment, the lacking item is one example of information (additional request information) to additionally be obtained from the patient. In obtaining the lacking item, the correspondence database (exemplary correspondence information) is referred to.

In the present embodiment, in step S90, instead of or in addition to the lacking item, information based on a request from a specialist may be presented. The information based on the request from the specialist is, for example, information of a type required by the specialist as to an infection of a type identified as the candidate and information of a type not included in the structured data (the electronic medical chart data read in step S30) generated in step S40. In this context, the information based on the request from the specialist is another example of the additional request information.

The type of the information required by the specialist may be stored in advance in the storage such as memory 200, for example, as a request correspondence table, for each type of the infection. In this context, the request correspondence table configures an example of information describing relation between a plurality of infections and the additional request information. In one implementation, when the estimation model estimates candidates for an infection, medical service support system 100 reads from the request correspondence table in the storage, the request from the specialist corresponding to the candidates for the infection. Of at least one type of information included in the read request, information of a type not included in the structured data generated in step S40 is then identified as the information based on the request from the specialist. The primary doctor can thus obtain in advance (before direct contact with the specialist), the type of the information requested by the specialist for the candidates for the infection. Therefore, the primary doctor can more promptly see the patient with the number of times of communication and/or a time period of communication with the specialist being reduced.

Identification of the information based on the request from the specialist may be performed in step S70 instead of or in addition to identification of the lacking item.

Identification of the information based on the request from the specialist may be performed by using an answer obtained by the primary doctor from the specialist in addition to or instead of reference to the request correspondence table described above. More specifically, the primary doctor may present to the specialist, the electronic medical chart data and candidates for the infection. In response, the specialist may request, in the answer to the primary doctor, information necessary for identification of the infection which may be affecting the patient. Medical service support system 100 may identify "necessary information" included in the answer from the specialist described above as the "information based on the request from the specialist." Medical service support system 100 may identify the "information based on the request from the specialist" by performing natural language processing on the answer from the specialist.

In one implementation, medical service support system 100 may accept an operation to transmit information to the specialist, in the display screen where information on candidates for an infection or the like is shown in step S90. Medical service support system 100 may transmit the electronic medical chart data and the candidates for the infection to an address associated with the specialist, in accordance with the operation. Thereafter, when medical service support system 100 receives the answer from the specialist, it may update the display screen such that the answer is added thereto. The answer from the specialist can thus be provided to the primary doctor. Information transmitted to the specialist may be the display screen generated in step S90. The patient information, the candidates for a disease, and a result of diagnosis by the primary doctor are thus transmitted to the specialist.

The medical service support system may present to the primary doctor, both of the information necessary for estimation of candidates for an infection by the estimation model and the request from the specialist. The primary doctor may obtain remarks for the patient with reference to the request from the specialist, and thereafter additionally input the remarks to the medical service support system. The medical service support system may use the information (remarks) additionally inputted as described above for estimation again by the estimation model. Contents of the "information necessary for estimation of candidates for an infection by the estimation model" may overlap with contents of "additionally inputted information" as described above. The medical service support system may present these two types of information to the primary doctor in a manner (for example, visually) distinguishable from each other or in the same manner.

### (Reference Data)

"Reference data" may be used as information to be referred to in identification of the lacking item in step S70. Information to be used for diagnosis or therapy for each disease is stored in the reference data. In step S70, medical service support system 100 identifies the lacking item by referring to the reference data for each of at least one candidate for the disease identified in step S50. In one implementation, medical service support system 100 identifies as the lacking item, a type of information not included in already obtained information, of types of information stored in the reference data in connection with the disease identified in step S50.

In the "information to be used for diagnosis or therapy," information to be used for diagnosis and information to be used for therapy may be distinguished from each other. For example, types of the information to be used for diagnosis in connection with the disease "pyelonephritis" include "fever," "frequent urination," "flank pain," and "age," and types of the information to be used for therapy include "anamnesis" and "age".

An instruction to provide information from information terminal 500 for which determination in step S20 is to be made may include a purpose (diagnosis or therapy) to provide information. In identification of the lacking item in step S70, medical service support system 100 may use any one of the information to be used for diagnosis or the information to be used for therapy, of the "information to be used for diagnosis or therapy" in accordance with the purpose included in the instruction. More specifically, when the purpose falls under "diagnosis", medical service support system 100 may use the information to be used for diagnosis, and when the purpose falls under "therapy", it may use the information to be used for therapy.

In the "information to be used for diagnosis or therapy," distinction between "essential information" and "optional information" may be made for information of at least one type. In the information ("fever," "frequent urination," "flank pain," and "age") to be used for diagnosis of the disease "pyelonephritis" described above, such distinction that "fever," "frequent urination," and "flank pain" are "essential information" and "age" is "optional information" may be made.

The lacking item identified with the use of the reference data may be outputted to the display screen described above in connection with step S90.

Furthermore, the display screen may include representation indicating whether or not the information already obtained by medical service support system 100 for the patient includes all of information of the types identified as "essential information" described above. Exemplary representation is an "OK" mark shown when the information already obtained by medical service support system 100 for the patient includes all types of "essential information" described above. Another example is an "NG" mark shown when the information already obtained by medical service support system 100 for the patient does not include at least one type of "essential information" described above.

Yet another exemplary representation may show a ratio of type(s) included in the information already obtained by medical service support system 100 for the patient to all types of "essential information" described above. In one example, representation of this ratio is a numerical value (100%, 50%, or the like). In another example where there are three types ("fever," "frequent urination," and "flank pain") of essential information, representation showing the ratio described above is "Good" when all of the three types are included, "OK" when one type or two types is/are included, and "NG" when none of the types is included.

### (Representation of Guideline)

In the display screen generated in step S90, medical service support system 100 may add information extracted from a guideline. In one implementation, medical service support system 100 adds to the display screen, a diagnosis guideline or a therapy guideline corresponding to the disease identified in step S50. An exemplary added guideline is JAID/JSC Guidelines for Clinical Management of Infectious Disease (https://www.kansensho.or.jp/uploads/files/guidelines/guideline_JAID-JSC_2015_urinary-tract.pdf).

Medical service support system 100 may extract a part corresponding to the identified disease from the guideline and add only the extracted part to the display screen. Alternatively, medical service support system 100 may add to the display screen, only one of an article of the guideline itself or information (for example, a URL) on access to the guideline. Medical service support system 100 may add to the display screen, information extracted from the guideline also similarly for a disease based on remarks made by the primary doctor.

Medical service support system 100 may select a type of the guideline to be added, in accordance with the purpose included in information provided from information terminal 500 for which determination in step S20 is to be made. More specifically, when the purpose falls under "diagnosis", medical service support system 100 adds information on the diagnosis guideline to the display screen, and when the purpose falls under "therapy", it may add information on the therapy guideline to the display screen.

### [Aspects]

Illustrative embodiments described above are understood by a person skilled in the art as specific examples of aspects below.

(Clause 1) A medical service support method according to one aspect may include obtaining at least one item of patient information relating to a patient, inputting the at least one item to an estimation model and obtaining candidates for a disease which may be affecting the patient from among a plurality of diseases, obtaining additional request information to additionally be obtained from the patient, based on the candidates for the disease, and presenting the additional request information.

According to the medical service support method in Clause 1, a non-specialist of a disease is provided with suggestion about a value of which item he/she should further consider, as additional request information. Support for medical service of the disease to the non-specialist of the disease is thus provided.

(Clause 2) In the medical service support method in Clause 1, the obtaining additional request information may include referring to reference data in which a disease and information to be used for diagnosis or therapy are associated with each other and identifying the additional request information based on the information to be associated with the candidates for the disease and to be used for diagnosis or therapy.

According to the medical service support method in Clause 2, information for studying appropriateness of the candidates for the disease and a method of therapy can be provided to a primary doctor.

(Clause 3) In the medical service support method in Clause 2, the reference data may include essential information and optional information as the information to be used for diagnosis or therapy, and the medical service support method may further include presenting a result of determination as to whether the at least one item includes all of the essential information.

According to the medical service support method in Clause 3, information for studying whether or not additional request information should actually be inputted can be provided to the primary doctor.

(Clause 4) In the medical service support method in Clause2 or 3, the information to be used for diagnosis or therapy may include diagnosis information and therapy information, the medical service support method may further include accepting diagnosis or therapy as a purpose of obtainment of the candidates for the disease, and the identifying the additional request information may include identifying the additional request information based on the diagnosis information when the purpose falls under diagnosis and identifying the additional request information based on the therapy information when the purpose falls under therapy.

According to the medical service support method in Clause 4, appropriate information can be provided to the primary doctor in accordance with a situation (therapy or diagnosis) that the primary doctor faces.

(Clause 5) The medical service support method in any one of Clauses 1 to 4 may further include showing a diagnosis guideline or a therapy guideline.

According to the medical service support method in Clause 5, more information on the disease can be provided to the primary doctor.

(Clause 6) The medical service support method in any one of Clauses 1 to 5 may further include accepting a result of diagnosis by a primary doctor together with the patient information and transmitting to a specialist, the patient information, the candidates for the disease, and the result of diagnosis by the primary doctor.

According to the medical service support method in Clause 6, more information can be given to a specialist and thus an answer from the specialist can be more appropriate.

(Clause 7) The medical service support method described in any one of Clauses 1 to 5 may further include showing as being emphasized, a basis of determination of the candidates for the disease by the estimation model.

According to the medical service support method in Clause 7, the basis for determination of inference which is a black box can be provided.

(Clause 8) In the medical service support method in any one of Clauses 1 to 7, the additional request information may include information that allows improvement in accuracy of the candidates for the disease when the information is inputted to the estimation model, and the obtaining additional request information may include obtaining the additional request information by referring to correspondence information in which each of the plurality of diseases is associated with items of the patient information.

According to the medical service support method in Clause 8, a step of obtaining additional request information is more specifically provided.

(Clause 9) In the medical service support method in any one of Clauses 1 to 8, the additional request information may include a lacking item not included in the at least one item among items of the patient information brought in correspondence with the candidates for the disease, and the obtaining additional request information may include identifying the lacking item.

According to the medical service support method in Clause 9, more specific information is provided as the additional request information.

(Clause 10) In the medical service support method in any one of Clauses 1 to 9, the obtaining additional request information may include obtaining the additional request information corresponding to the obtained candidates for the disease from a storage where information describing relation between the plurality of diseases and the additional request information is stored or obtaining the additional request information by using an answer obtained by presenting the obtained candidates for the disease to a specialist.

According to the medical service support method in Clause 10, more specific information is provided as the additional request information.

(Clause 11) In the medical service support method in any one of Clauses 1 to 10, the obtaining a value of at least one item may include accepting input of electronic medical chart data of the patient and generating the value of the at least one item by using the electronic medical chart data.

According to the medical service support method in Clause 11, the non-specialist should only input medical chart data of a patient in order to be provided with information, instead of inputting the value of at least one item. Therefore, operations performed by the non-specialist are facilitated.

(Clause 12) The medical service support method in any one of Clauses 1 to 11 may further include presenting the candidates for the disease, and the presenting the candidates for the disease may include presenting an item having importance equal to or higher than a given value in identification of the candidates for the disease, of the at least one item, or additional information on the candidates for the disease.

According to the medical service support method in Clause 12, in connection with the presented candidates, information useful for understanding the reason why the candidates are presented is provided.

(Clause 13) In the medical service support method in any one of Clauses 1 to 12, the patient information may include information on at least one of patient interview information, physical finding, and an examination result.

According to the medical service support method in Clause 10, candidates for an infection which may be affecting a patient are provided by input of information available to the primary doctor of the patient.

(Clause 14) A medical service support program according to one aspect, by being executed by at least one processor of a computer, may cause the computer to perform the medical service support method in any one of Clauses 1 to 13.

According to the medical service support program in Clause 14, a non-specialist of a disease is provided with suggestion about a value of which item he/she should further consider, as additional request information. Support for medical service of the disease to the non-specialist of the disease is thus provided.

(Clause 15) A medical service support system according to one aspect includes a processor, a communication interface, and a storage where an estimation model is stored, the estimation model being configured to output candidates for a disease which may be affecting a person associated with patient information among a plurality of diseases, in response to input of at least one item of the patient information, the processor may obtain input of a value of the at least one item of the patient information relating to a patient, input the value of the at least one item to the estimation model to obtain candidates for the disease which may be affecting the patient, and obtain additional request information to additionally be obtained from the patient, based on the candidates for the disease, and the communication interface may present the additional request information.

According to the medical service support system in Clause 15, a non-specialist of a disease is provided with suggestion about a value of which item he/she should further consider, as additional request information. Support for medical service of the disease to the non-specialist of the disease is thus provided.

Though the embodiments of the present invention have been described, it should be understood that the embodiments disclosed herein are illustrative and non-restrictive in every respect. The scope of the present invention is defined by the terms of the claims and is intended to include any modifications within the scope and meaning equivalent to the terms of the claims. It is intended that each technique in the embodiment can be carried out alone or in combination to the extent possible with another technique in the embodiment as necessary.

### REFERENCE SIGNS LIST

100 medical service support system; 101 processor; 500 information terminal; 600, 601, 602, 603, 604 screen; 610 first communication frame; 611, 612, 621, 622, 623, 624, 625, 626, 627, 630 field; 619 button; 640 second communication frame.

## Claims

1. A medical service support method comprising:
obtaining at least one item of patient information relating to a patient;
inputting the at least one item to an estimation model and obtaining candidates for a disease which may be affecting the patient from among a plurality of diseases;
obtaining additional request information to additionally be obtained from the patient, based on the candidates for the disease; and
presenting the additional request information.

2. The medical service support method according to claim 1, wherein
the obtaining additional request information includes:
referring to reference data in which a disease and information to be used for diagnosis or therapy are associated with each other; and
identifying the additional request information based on the information to be associated with the candidates for the disease and to be used for diagnosis or therapy.

3. The medical service support method according to claim 2, wherein
the reference data includes essential information and optional information as the information to be used for diagnosis or therapy, and
the medical service support method further comprises presenting a result of determination as to whether the at least one item includes all of the essential information.

4. The medical service support method according to claim 2, wherein
the information to be used for diagnosis or therapy includes diagnosis information and therapy information,
the medical service support method further comprises accepting diagnosis or therapy as a purpose of obtainment of the candidates for the disease, and
the identifying the additional request information includes:
identifying the additional request information based on the diagnosis information when the purpose falls under diagnosis; and
identifying the additional request information based on the therapy information when the purpose falls under therapy.

5. The medical service support method according to claim 1, further comprising showing a diagnosis guideline or a therapy guideline for the candidates for the disease.

6. The medical service support method according to claim 1, further comprising:
accepting a result of diagnosis by a primary doctor together with the patient information; and
transmitting to a specialist, the patient information, the candidates for the disease, and the result of diagnosis by the primary doctor.

7. The medical service support method according to claim 1, further comprising showing as being emphasized in the patient information, a basis of determination of the candidates for the disease by the estimation model.

8. The medical service support method according to claim 1, wherein
the additional request information includes information that allows improvement in accuracy of the candidates for the disease when the information is inputted to the estimation model, and
the obtaining additional request information includes obtaining the additional request information by referring to correspondence information in which each of the plurality of diseases is associated with items of the patient information.

9. The medical service support method according to claim 1, wherein
the additional request information includes a lacking item not included in the at least one item among items of the patient information brought in correspondence with the candidates for the disease, and
the obtaining additional request information includes identifying the lacking item.

10. The medical service support method according to claim 1, wherein.
the obtaining additional request information includes:
obtaining the additional request information corresponding to the obtained candidates for the disease from a storage where information describing relation between the plurality of diseases and the additional request information is stored; or
obtaining the additional request information by using an answer obtained by presenting the obtained candidates for the disease to a specialist.

11. The medical service support method according to claim 1, wherein
the obtaining at least one item includes:
accepting input of electronic medical chart data of the patient: and
generating a value of the at least one item by using the electronic medical chart data.

12. The medical service support method according to claim 1, further comprising presenting the candidates for the disease, wherein
the presenting the candidates for the disease includes presenting an item having importance equal to or higher than a given value in identification of the candidates for the disease, of the at least one item, or additional information on the candidates for the disease.

13. The medical service support method according to claim 1, wherein
the patient information includes information on at least one of patient interview information, physical finding, and an examination result.

14. A medical service support program, by being executed by at least one processor of a computer, causing the computer to perform the medical service support method according to claim 1.

15. A medical service support system comprising:
at least one processor;
a communication interface; and
a storage where an estimation model is stored, the estimation model being configured to output candidates for a disease which may be affecting a person associated with patient information among a plurality of diseases, in response to input of at least one item of the patient information, wherein
the processor
obtains input of a value of the at least one item of the patient information relating to a patient,
inputs the value of the at least one item to the estimation model to obtain candidates for the disease which may be affecting the patient, and
obtains additional request information to additionally be obtained from the patient, based on the candidates for the disease, and
the communication interface presents the additional request information.
